# EUROPÄISCHE PATENTSCHRIFT

(11) **EP 2 314 342 B1**
(45) Veröffentlichungstag und Bekanntmachung des Hinweises auf die Patenterteilung: **27.06.2012**
(21) Anmeldenummer: 09405186.9
(22) Anmeldetag: 23.10.2009
(51) Int. Cl.: A61M 37/00

(54) **Vorrichtung zum Injizieren eines Feststoffes**
Device for injecting a solid substance
Dispositif d'injection d'une matière solide

(43) Veröffentlichungstag der Anmeldung: 27.04.2011
(73) Patentinhaber: forteq Nidau AG, 2560 Nidau (CH)
(72) Erfinder: Schmalz, Christian, CH-3250 Lyss (CH)
(74) Vertreter: Rüfenacht, Philipp Michael

(56) Entgegenhaltungen:
- US-A1- 2009 182 267

## Beschreibung

### Technisches Gebiet

Die Erfindung betrifft eine Vorrichtung zum Injizieren eines Feststoffes in einen menschlichen oder tierischen Körper, umfassend einen Spritzenkörper mit einem ersten rohrförmigen Innenraum zur Aufnahme des Feststoffes, eine Rückhaltevorrichtung zum Zurückhalten des Feststoffes im Spritzenkörper, eine Kanüle mit einem zweiten rohrförmigen Innenraum, wobei die Kanüle mit dem Spritzenkörper verbunden ist, und wobei der zweite rohrförmige Innenraum der Kanüle mit dem ersten rohrförmigen Innenraum des Spritzenkörpers kommuniziert, und einen Stössel, welcher durch den ersten Innenraum hindurch in den zweiten Innenraum verfahrbar ist. Die Erfindung betrifft weiter ein Verfahren zum Injizieren eins Feststoffes.

### Stand der Technik

Die Injizierung von Feststoffen ist ein Standardverfahren in der Human- und Veterinärmedizin und stellt eine Alternative zu den anderen Applikationsverfahren dar (z.B. oral, intravenös etc.). Dabei wird ein Feststoff mittels einer Injektionsvorrichtung in den menschlichen oder tierischen Körper eingeführt. Typischerweise handelt es sich bei den Feststoffen entweder um eine Markierung (zum Beispiel zur Identifikation und/oder zur Überwachung von Tieren) oder um ein Feststoffmedikament. Der Einsatz von Feststoffmedikamenten ist sowohl bei Tieren wie auch bei Menschen üblich und hat den Vorteil, dass durch die langsame Resorption des Wirkstoffes das Medikament in tieferer Frequenz verabreicht werden kann, womit entsprechend weniger Arztbesuche notwendig sind. Anwendungsgebiete der Injizierung von Feststoffen finden sich unter anderem in der Hormon- und der Schmerztherapie.

Typischerweise erfolgt die Injektion des Feststoffes mittels einer Feststoffspritze, welche im Wesentlichen einen Spritzenkörper mit einer Kanüle und einen Stössel umfasst. Nach dem Einführen der Kanüle in den Körper wird der Feststoff mittels des Stössels in die Kanüle geführt. Anschliessend wird bei ortsfestem Stössel die Kanüle aus dem Körper hinausgezogen. Der Feststoff wird dabei durch den Stössel im Körper zurückgehalten. Schliesslich wird der Stössel aus dem Körper gezogen.

Die DE 37 45 071 C2 (Bio Medic Data Systems) zeigt eine Nadelanordnung zum Markieren von Tieren. Die Vorrichtung umfasst ein Hohlrohr mit einer Austrittsöffnung und einer Eintrittsöffnung, sowie einer Treibstifteinrichtung. Die Seite des Hohlrohres mit der Eintrittsöffnung ist in einen Stöpsel eingeformt, welcher sich entlang eines Teils der Rohrlänge erstreckt. Ein Stift mit einer Dichtungsscheibe dient dazu, die Markierung in der Nähe der Austrittsöffnung im Hohlrohr anzuordnen. Ein einstückig mit der Hülse ausgebildeter Vorsprung verläuft durch die Öffnung. Dieser nimmt die Markierung in Reibeingriff, bis eine Kraft an einem Kolben und darüber wiederum an der Markierung liegt, die ausreicht um letztere durch das Rohr zu drücken. Ein Herausfallen der Markierung aus dem Hohlrohr vor dem Einpflanzen wird durch eine Beschichtung der Aussenfläche der Markierung verhindert.

Die US 5,484,403 (Avid Marketing) offenbart eine Spritze zum Implantieren von festen Objekten unter die Haut. Die Spritze besteht aus einem Zylinder, einer mit dem Zylinder verbundenen Kanüle und einem Stössel. Die Kanüle weist Haltevorrichtungen auf, um das zu implantierende Objekt bis zur Implantation zu halten, nämlich gefaltete Bereiche der Kanülenwand. Alternativ kann auch ein Einschnitt in der Kanüle vorgesehen sein, wobei der Durchmesser der Kanüle geringer als derjenige des Objekts ist.

Die EP 0 639 387 A (Texas Instruments Inc.) zeigt einen Injektor zum Einführen von festen Objekten, wie zum Beispiel Sendern, in ein Lebewesen, wobei der Injektor einen Zylinder zur Aufnahme eines Objektes und einen darin verschiebbaren Stössel zum Ausstossen des Objektes aufweist. Im Zylinder sind Klemmvorrichtungen zum mechanischen Halten des Objektes vorgesehen. Die Klemmvorrichtungen sind als federnde Elemente ausgebildet. Das federnde Element besteht aus einer Lippe aus federndem Material und ist parallel zur Längsachse des Zylinders angeordnet. Das Ausstossen des Objektes erfolgt mittels des Stössels, wobei letzterer einen geringeren Durchmesser hat als das Objekt, so dass dessen axiale Bewegung von der Lippe nicht beeinflusst ist.

Eine weitere aus dem Stand der Technik bekannte Vorrichtung zum Injizieren eines Feststoffes ist im Dokument US 2009/182267 A1 offenbart. Der Oberbegriff des Anspruchs 1 ist auf diesem Dokument basiert.

Die gemäss dem Stand der Technik bekannten Vorrichtungen zum Injizieren von Feststoffen haben den Nachteil, dass insbesondere fragile Feststoffe bei der Injektion beschädigt werden können.

### Darstellung der Erfindung

Aufgabe der Erfindung ist es, eine dem eingangs genannten technischen Gebiet zugehörende Vorrichtung zum feststoffschonenden Injizieren eines Feststoffes zu schaffen.

Die Lösung der Aufgabe ist durch die Merkmale des Anspruchs 1 definiert. Gemäss der Erfindung ist der Stössel direkt mit der Rückhaltevorrichtung zusammenwirkbar ausgebildet, so dass die Rückhaltevorrichtung mittels des Stössels deaktivierbar ist.

Dadurch ergibt sich der Vorteil, dass vor der Injektion des Feststoffes, insbesondere vor einer Verschiebung des Feststoffes im Spritzenkörper die Rückhaltevorrichtung deaktiviert werden kann, so dass beim Verschieben des Feststoffes die Haltevorrichtung mit Ausnahme der üblichen Normalkräfte keine radiale Kraftkomponente, welche rechtwinklig zur Förderrichtung des Feststoffes gerichtet ist, auf den Feststoff ausübt. Bei der Verwendung der Vorrichtung wird der Feststoff während des Verfahrens durch den Spritzenkörper und die Kanüle auch keiner radialen Kraftkomponente einer Rückhaltevorrichtung ausgesetzt, womit der Feststoff schonend zum Beispiel in einen menschlichen Körper injiziert werden kann. Damit können auch Feststoffe injiziert werden, welche bis zu einem gewissen Grad spröde oder deformierbar sind. Auch Kapseln, welche zum Beispiel Flüssigkeiten enthalten, können damit injiziert werden, ohne dass ein Aufbrechen aufgrund einer Quetschung der Kapsel durch die Rückhaltevorrichtung droht. Weiter wird dadurch eine besonders ergonomische und wenig fehleranfällige Spritzenbedienung erreicht, da die Deaktivierung der Rückhaltevorrichtung mit derselben Bewegung erfolgt wie die Injektion des Feststoffes.

Der Spritzenkörper ist vorzugsweise im Wesentlichen als länglicher Hohlzylinder ausgebildet. Der Hohlzylinder hat typischerweise einen kreiszylindrischen Innenraum. Die Kanüle kann aus Metall, insbesondere Stahl ausgebildet sein, wobei aber auch Kunststoffe eingesetzt werden können. Die Kanüle ist bevorzugt am distalen Ende abgeschrägt und an einem vordersten Ende als Spitze ausgeformt, wobei die an die Spitze angrenzenden Kanten auch scharf sein können, so dass ein Eindringen in einen menschlichen oder tierischen Körper vereinfacht wird. Das proximale Ende der Kanüle ist mit dem Spritzenkörper so verbunden, dass der erste Innenraum des Spritzenkörpers mit dem Innenraum der Kanüle kommuniziert. Die Verbindung zwischen Spritzenkörper und Kanüle erfolgt bevorzugt über eine Verpressung oder durch Verkleben. Zum Schutz der Kanüle und des Benutzers kann eine Schutzkappe vorgesehen sein, welche vor der Verwendung über die Kanüle führbar ist und form- und/oder kraftschlüssig gehalten wird. Weiter kann ein form- und/oder kraftschlüssig mit der Spritze verbundenes Sicherheitselement vorgesehen sein, welches in montiertem Zustand verhindert, dass der Stössel unbeabsichtigt in den Spritzenkörper geschoben wird. Dieses kann als längliches Element ausgebildet sein, welches unterhalb des Stösselkopfes am Stössel mittels Clipverbindung gehalten wird und so zwischen dem Spritzenkörper und dem Stösselkopf den notwendigen Abstand sichert.

Der Stössel umfasst einen Kolben, einen dem Kolben gegenüberliegenden Stösselkopf, wo der Stössel bedient wird, sowie eine Verbindung zwischen dem Kolben und dem Stösselkopf, welche typischerweise als länglicher Stab mit einem kleineren Durchmesser als der Kolben ausgebildet ist. Gegebenenfalls kann auch auf die Verbindung verzichtet werden, womit der Stössel lediglich einen Kolben und einen Stösselkopf umfasst. Dies ist dann von Vorteil, wenn der Durchmesser des Kolbens sehr klein ist, insbesondere in einem Millimeterbereich oder darunter. Damit wird die Stabilität des Stössels erhöht.

Die Injektion eines Feststoffes mit der erfindungsgemässen Spritze erfolgt vorzugsweise gemäss den nachfolgenden Schritten:
a) Die Kanüle wird in den menschlichen oder tierischen Körper eingeführt.
b) Mittels des Stössels wird die Rückhaltevorrichtung deaktiviert und der Feststoff in die Kanüle überführt.
c) Der Spritzenkörper wird bei bezüglich des Körpers ortsfestem Stössel zurückgezogen, so dass der Feststoff ausserhalb der Kanüle im Körper verbleibt.

Vorzugsweise umfasst die Rückhaltevorrichtung ein Rückhalteelement, wobei der Stössel bei der Deaktivierung der Rückhaltevorrichtung vom Rückhalteelement beabstandet ist. Der zu injizierende Feststoff ist vor der Verwendung der Vorrichtung distal vor dem Stössel platziert. Das Rückhalteelement ist so platziert, dass es mit dem Feststoff zusammenwirken kann. Das Rückhaltelement kann nun mittels Fernwirkung durch den Stössel deaktiviert werden. Damit kann die Rückhaltevorrichtung vor der Injektion deaktiviert werden, womit der Feststoff nicht entgegen einer Reibungskraft verschoben werden muss, durch welche der Feststoff beschädigt werden könnte. Dies hat den Vorteil, dass der Benutzer nicht an die Deaktivierung der Rückhaltevorrichtung denken muss, sondern einfach den Stössel durch den Spritzenkörper führen kann. Dazu können verschiedenste Mittel vorgesehen sein:

Der Stössel kann seitlich fest mit einem länglichen Abschnitt federnd verbunden sein, welcher über den Stössel in distaler Richtung hinausragt. Am distalen Ende des länglichen Abschnittes kann eine Nase vorgesehen sein, welche in einem ersten Zustand durch eine Öffnung in den Innenraum des Spritzenkörpers hineinragt und so den Feststoff zurückhält oder per Reibschluss festklemmt. Weiter weist bei dieser Variante der Spritzenkörper unmittelbar hinter der Nase (in distaler Richtung) am Öffnungsrand eine Rampe auf. Beim Verfahren des Stössels in distaler Richtung wird der längliche Abschnitt über die Rampe aus dem Innenraum des Spritzenkörpers gehoben und gibt so den Weg für den Feststoff frei. Der längliche Abschnitt muss dabei natürlich nicht unbedingt mit dem Stössel fest verbunden sein. Der längliche Abschnitt kann auch zwangsgeführt sein, wobei der Stössel zum Beispiel eine Nase aufweist, welche mit diesem zusammenwirken kann. Damit erfolgen die Deaktivierung und die Injektion mit derselben Bewegung.

Ähnlich wie bei der obig beschriebenen Variante kann die Nase über eine bezüglich des Spritzenkörperinnenraums tangential verlaufende Rampe, mittels einer Rotation des Stössels um dessen Achse, aus dem Innenraum des Spritzenkörpers verfahren werden. Vor der Injektion müsste als die Rückhaltevorrichtung mittels einer Rotation des Stössels deaktiviert werden.

Das Rückhalteelement kann auch über Zugmittel mit dem Stössel verbunden sein, so dass vor der Injektion der Stössel in proximaler Richtung verfahren wird, womit das Rückhaltelement aus dem Spritzenkörper hinausgezogen wird und beim Verfahren des Stössels in distaler Richtung, beim injizieren des Feststoffes, nicht mehr in den Spritzenkörper gelangt. Dies bedeutet jedoch, dass für die Injektion zwei verschiedene Bewegungen ausgeführt werden müssen.

Vorzugsweise ist der Feststoff vor der Deaktivierung der Rückhaltevorrichtung zwischen dem Stössel und dem Rückhalteelement gehalten. Damit wird erreicht, dass der Feststoff während der Lagerung der Vorrichtung nicht durch eine von der Rückhaltevorrichtung verursachten Kraft beaufschlagt und damit schonend gelagert ist. Das Rückhalteelement schliesst dazu in aktiviertem Zustand mindestens teilweise den Querschnitt des Innenraums des Spritzenkörpers distal vor dem Feststoff. Bei deaktivierter Rückhaltevorrichtung schliesst das Rückhalteelement den Querschnitt nicht, respektive nur soweit, dass der Feststoff am Rückhalteelement vorbeigeschoben werden kann. Alternativ kann das Rückhalteelement den Feststoff seitlich mittels Reibschluss an einem unbeabsichtigten Hinausrutschen aus dem Spritzenkörper, respektive aus der Kanüle hindern.

Bevorzugt ist die Rückhaltevorrichtung einstückig mit dem Spritzenkörper ausgebildet. Dadurch wird ein einfach aufgebauter Spritzenkörper mit Rückhaltevorrichtung geschaffen, welcher kostengünstig hergestellt werden kann. Der Spritzenkörper kann dabei auch mehrere Teile umfassen, wobei aber bevorzugt der Spritzenkörper und die Rückhaltevorrichtung als ein Bauteil ausgebildet sind.

Alternativ kann die Rückhaltevorrichtung auch als eigenes Bauteil oder sogar mehrteilig aufgebaut sein. Dies hat den Vorteil, dass zum Beispiel Spritzgussformen für die Produktion der Rückhaltevorrichtung und den Spritzenkörper einfacher gestaltet sein können. Anderseits müssen beim Zusammenbauen der Spritze mehr Verfahrensschritte in Kauf genommen werden.

Das Rückhalteelement ist bevorzugt als mindestens eine in den ersten Innenraum des Spritzenkörpers eindringende erste Nase ausgebildet. Mit der ersten Nase kann der Feststoff vor der Injektion im Spritzenkörper zurückgehalten werden. Die Rückhaltung kann zum Beispiel so erfolgen, dass der Feststoff proximal hinter der Nase angeordnet ist und so am Hinausrutschen gehindert wird. Weiter kann die Nase auch seitlich mittels Reibschluss auf den Feststoff einwirken und so denselben an einem Hinausrutschen hindern. Schliesslich könnte der Feststoff auch eine Vertiefung aufweisen, in welche die Nase eindringen kann und damit ein Verrutschen des Feststoffes in beide axiale Richtungen verhindert. Damit könnte auch eine Rückhaltevorrichtung geschaffen werden, bei welcher keine radialen Kraftkomponenten auf den Feststoff wirken. Der Feststoff müsste dazu aber entsprechend geformt werden.

Statt einer Nase kann auch ein dem Querschnitt des ersten Innenraums entsprechendes Rückhalteelement vorgesehen sein, welches den Querschnitt vollständig verschliesst. Dies kann dann von Vorteil sein, wenn der Feststoff besonders instabil ist. Das Rückhalteelement kann in diesem Fall auch den Raum, in dem der Feststoff angeordnet ist weitgehend luftdicht abschliessen. Dabei ist jedoch der Weg, den das Rückhalteelement zurücklegen muss, um den ersten Innenraum für den Feststoff freizugeben, grösser.

Vorzugsweise ist die erste Nase federnd mit dem Spritzenkörper verbunden. Dadurch wird eine besonders einfache Realisierung der Rückhaltevorrichtung erreicht. In einem ersten Zustand, in welchem die Federung entspannt ist, ragt die erste Nase in den ersten Innenraum des Spritzenköpers ein. In einem zweiten Zustand, bei einer Kraftbeaufschlagung der Nase in radialer Richtung, ragt die erste Nase teilweise oder nicht in den ersten Innenraum des Spritzenkörpers ein.

Die federnde Verbindung kann auf verschiedenste Arten realisiert sein:
1. Die Nase kann mittels einer Schrauben- oder Kegelfeder, welche radial zur Längsrichtung des Spritzenkörpers orientiert ist, angeordnet sein und so die Nase in den ersten Innenraum pressen.
2. Die Nase kann mittels einer Plattfeder, welche axial oder um den Spritzenkörper teilweise umlaufend angeordnet ist ("Teilkreisspiralfeder"), mit einer Kraft beaufschlagt werden.
3. Die Nase kann mittels eines elastischen Rings um den Spritzenkörper mit einer Kraft beaufschlagt werden, wobei der elastische Ring zum Beispiel als Gummiband oder ähnliches ausgebildet ist.
4. Schliesslich kann die Nase selbst als elastisches Element ausgebildet sein.

Die Nase kann zur Deaktivierung der Rückhaltevorrichtung sowohl über das federnde Element wie auch direkt über die Nase selbst aus dem ersten Innenraum geführt werden.

Alternativ oder zusätzlich kann auch eine Einrastung der Nase oder eine reibschlüssige Haftung der Nase vorgesehen sein, welche mittels des Stössels deaktivierbar ist. Auch eine Perforation im Bereich der Nase, welche durch eine radial gerichtete Kraft gebrochen wird, kann als alternative Realisierung zur federnden Rückhaltevorrichtung vorgesehen sein, jedenfalls dann, wenn die Vorrichtung für den einmaligen Gebrauch vorgesehen ist. Bevorzugt umfasst die Rückhaltevorrichtung einen länglichen Abschnitt, welcher an einem distalen Ende mit der ersten Nase verbunden ist und an einem proximalen Ende insbesondere federnd und/oder verschwenkbar mit dem Spritzenkörper verbunden ist, wobei in einem ersten Zustand die Nase in den ersten Innenraum des Spritzenkörpers eindringt und in einem zweiten Zustand höchstens teilweise in den ersten Innenraum des Spritzenkörpers eindringt, insbesondere nicht in den ersten Innenraum des Spritzenkörpers eindringt. Damit wird eine einfache und kostengünstige Realisierung der Rückhaltevorrichtung erreicht. Der längliche Abschnitt kann dazu selbst aus einem federnden Material bestehen, oder am proximalen Ende verschwenkbar und mittels einer Feder beaufschlagt ausgebildet sein. In einem ersten Zustand kann der Feststoff hinter der Nase angeordnet sein. Der Feststoff kann auch im Bereich der Nase angeordnet und zum Beispiel durch Reibschluss durch die Nase gehalten sein.

Alternativ kann statt des länglichen Abschnittes auch ein Tubus vorgesehen sein, welcher zum Beispiel aus einem flexiblen, insbesondere elastischen Material besteht.

Vorzugsweise umfasst die Rückhaltevorrichtung zwei bezüglich des ersten Innenraums gegenüberliegende längliche Abschnitte, welche am distalen Ende mit Nasen verbunden sind und wobei die länglichen Abschnitte am proximalen Ende insbesondere federnd und/oder verschwenkbar mit dem Spritzenkörper verbunden sind. Damit wird ein Risiko des Verkanntens des Feststoffes verringert.

Alternativ kann auch nur ein länglicher Abschnitt vorgesehen sein, womit aber das Risiko des Verkanntens erhöht wird. Weiter können auch mehr als zwei längliche Abschnitte vorgesehen sein, wobei bevorzugt eine symmetrische Anordnung um den ersten Innenraum gewählt wird. Dies bedeutet aber einen erhöhten konstruktiven Aufwand und damit höhere Herstellungskosten.

Bevorzugt ist der längliche Abschnitt zwischen einer in Längsrichtung orientierten U-förmigen Ausnehmung im Spritzenkörper gebildet. So kann der Spritzenkörper mit der Rückhaltevorrichtung auf einfache Weise aus einem Stück gebildet werden, womit ein besonders effizientes und kostengünstiges Herstellungsverfahren erreicht werden kann. Weiter kann dadurch ein besonders kompakter Spritzenkörper mit einer Haltevorrichtung gebildet werden. Der Spritzenkörper kann zum Beispiel mittels einer entsprechenden Gussform so hergestellt werden, dass die U-förmige Ausnehmung schon ausgebildet ist. Anderseits kann die U-förmige Ausnehmung mittels eines Stanzvorgangs oder einer Fräsung nach der Herstellung des Spritzenkörpers erfolgen.

Alternativ kann der längliche Abschnitt auch separat hergestellt und mit dem Spritzenkörper verbunden werden. Damit sind aber ein erhöhter konstruktiver Aufwand und damit Mehrkosten verbunden.

Der längliche Abschnitt ist bevorzugt im ersten Zustand im Wesentlichen parallel zu einer Längsrichtung des ersten Innenraums des Spritzenkörpers orientiert und im zweiten Zustand zur Längsrichtung des ersten Innenraums verschwenkt orientiert. Bei einer Ausbildung des länglichen Abschnittes mittels einer U-förmigen Ausnehmung im Spritzenkörper ergibt sich in besonders einfacher Weise ein länglicher Abschnitt, welcher parallel zur Längsrichtung des ersten Innenraums orientiert ist und welcher bei Kraftbeaufschlagung verschwenkt werden kann. Die Verschwenkung erfolgt dabei bevorzugt bezüglich des ersten Innenraums radial nach aussen. Der längliche Abschnitt ist dabei bevorzugt elastisch, respektive federnd bezüglich des Spritzenkörpers ausgebildet, was insbesondere durch eine geeignete Materialwahl erreicht werden kann. Typischerweise ist die Haltevorrichtung im ersten Zustand aktiv und im zweiten Zustand nicht aktiv. Gegebenenfalls kann aber die Haltevorrichtung auch im zweiten Zustand aktiv und im ersten Zustand nicht aktiv sein, insbesondere, wenn eine Schwenkachse radial nach aussen vom ersten Innenraum beabstandet ist.

Alternativ kann der längliche Abschnitt auch radial, teilweise um den ersten Innenraum umlaufend angeordnet sein. Dadurch kann die Stabilität des Spritzenkörpers beeinträchtigt werden.

Bevorzugt weist der längliche Abschnitt im Bereich des proximalen Endes eine zweite Nase auf, welche im ersten Zustand in den Innenraum des Spritzenkörpers eindringt und im zweiten Zustand höchstens teilweise in den ersten Innenraum des Spritzenkörpers eindringt, insbesondere nicht in den ersten Innenraum des Spritzenkörpers eindringt, wobei der Feststoff im ersten Zustand zwischen der ersten Nase und der zweiten Nase zurückgehalten ist und im zweiten Zustand nicht zurückgehalten ist. Damit wird eine Rückhaltevorrichtung geschaffen, welche den Feststoff an einer Bewegung in beide axialen Richtungen hindert und örtlich fixieren kann. Typischerweise ist der Abstand zwischen den Nasen so bemessen, dass der Feststoff dazwischen gehalten ist. Dazu weist der Abstand eine Länge auf, welche die Länge des Feststoffes geringfügig, insbesondere um weniger als 10%, bevorzugt weniger als 5% übertrifft. Damit wird erreicht, dass der Feststoff nicht zwischen den Nasen eingeklemmt oder durch die Nasen beschädigt werden kann. Gleichzeitig wird aber auch eine axiale Hin- und Herbewegung des Feststoffs verhindert, welche beim Anschlagen an den Nasen ebenfalls zu Beschädigungen führen könnte. Der Abstand der Nasen kann weiter so ausgelegt werden, dass bei gegebener Längentoleranz des Feststoffes bei der Produktion der Vorrichtung zum Beispiel 99%, oder 99,9% der Feststoffe zwischen den Nasen gehalten werden können, d.h. dass 99% oder 99,9 % der Feststoffe eine geringere Länge als der Abstand zwischen den Nasen aufweisen. Die Nasen können aber auch einen grösseren Abstand aufweisen, so dass produktionsbedingte Komplikationen beim Einbringen des Feststoffes zwischen den Nasen weitgehend unterbunden werden können. Die Nasen können aber auch einen geringeren Abstand als die Länge des Feststoffes aufweisen, wobei der Feststoff zum Beispiel seitlich, mittels Reibschluss gehalten wird.

Alternativ kann auf die zweite Nase auch verzichtet werden, insbesondere wenn zum Beispiel der längliche Abschnitt mit dem Stössel verbunden ist, wobei durch das Verfahren des Stössels in distaler Richtung die erste Nase über eine Rampe aus dem ersten Innenraum des Spritzenkörpers ausgefahren wird. In diesem Fall wird der Feststoff durch den Stössel an einer Bewegung in proximaler Richtung gehindert.

Der Stössel und/oder die zweite Nase weisen vorzugsweise in proximaler Richtung eine Abschrägung auf, so dass bei einem Zusammenwirken des Stössels mit der zweiten Nase zumindest eine Kraftkomponente radial auf die zweite Nase beaufschlagbar und der längliche Abschnitt in den zweiten Zustand überführbar ist. Die so ausgebildete zweite Nase kann zwei Funktionen übernehmen, nämlich das Zurückhalten des Feststoffes bezüglich der proximalen Richtung und die Funktion eines Hebelarmes, um den länglichen Abschnitt zu verschwenken. Nach dem Verschwenken des länglichen Abschnittes verfährt der Stössel weiter in distaler Richtung, um den Feststoff durch die Kanüle zu befördern. Um dies zu ermöglichen, weist die zweite Nase eine Abschrägung auf. Die Abschrägung kann dabei eine konstante Steigung aufweisen. Die Frontfläche des Stössels schliesst beim Kontaktieren der Abschrägung zu derselben in einem ersten Moment einen Winkel ein, welcher sich beim weiteren Verfahren des Stössels in distaler Richtung vergrössert. Während des Verfahrens des Stössels nimmt dabei die aufzuwendende Kraft ab , da der Winkel zwischen der Frontfläche des Stössels und der Abschrägung und auch der Hebel, welcher zwischen dem Kontaktbereich des Stössels an der Abschrägung und dem Verschwenkungspunkt liegt, vergrössert wird. Um eine gleichmässige Kraftaufwendung zu erreichen, kann die Abschrägung auch konkav ausgebildet sein, womit der grösser werdende Hebel durch die zunehmende Steigung der Abschrägung im Wesentlichen kompensiert werden kann. Dadurch wird erreicht, dass kein ruckartiges Durchstossen des Stössels erfolgt und damit die Vorrichtung in der Handhabung sicherer ist.

Alternativ kann auch auf die Abschrägung verzichtet werden, insbesondere wenn die zweite Nase eine geringe radial bemessene Höhe aufweist.

Bevorzugt ist eine radial bemessene Höhe der ersten Nase grösser als eine radial bemessene Höhe der zweiten Nase. Damit kann eine Optimierung der Verschwenkung des länglichen Abschnittes erreicht werden. Die zweite Nase wird durch den Stössel bis maximal in den Randbereich des ersten Innenraums gedrückt. Dabei bewegt sich die erste Nase über den länglichen Abschnitt gleichzeitig aus dem ersten Innenraum hinaus. Falls die zweite Nase dieselbe Höhe wie die erste Nase aufweist wird die erste Nase weiter als notwendig radial nach aussen geführt. Damit wird mehr Kraft als notwendig für die Deaktivierung der Rückhaltevorrichtung aufgewendet. Weiter kann dadurch die Vorrichtung während der Verwendung weniger kompakt sein. Um diese Bewegungen zu optimieren, können die Nasen so dimensioniert werden, dass die zweite Nase im deaktivierten Zustand knapp nicht in den ersten Innenraum des Spritzenkörpers ragt. Der Abstand zwischen der ersten Nase und dem ersten Innenraum ist bei deaktivierter Rückhaltevorrichtung bevorzugt minimal.

Vorzugsweise liegt ein Verhältnis zwischen der radial bemessenen Höhe der ersten Nase zu einem Abstand der ersten Nase zum proximalen Ende des länglichen Abschnittes in einem Bereich eines Verhältnisses zwischen der radial bemessenen Höhe der zweiten Nase zu einem Abstand der zweiten Nase zum proximalen Ende des länglichen Abschnittes. Damit wird eine optimale Grössenverteilung der beiden Nasen erreicht. Wenn nämlich durch den Stössel die zweite Nase aus dem ersten Innenraum des Spritzenkörpers hinausgeschoben ist, wird zugleich die erste Nase aus dem ersten Innenraum des Spritzenkörpers hinausgeschoben. Um aber allfälligen Bauungenauigkeiten des Spritzenkörpers und der Rückhaltevorrichtung vorzubeugen, wird bevorzugt die erste Nase bezüglich des obig beschriebenen Verhältnisses geringfügig kleiner ausgebildet, insbesondere ungefähr 5 - 10% kleiner als die theoretisch optimale Höhe.

Alternativ können die erste und die zweite Nase auch dieselbe Höhe aufweisen, insbesondere, wenn die Nasen eine sehr kleine Höhe aufweisen oder der längliche Abschnitt relativ kurz ist.

Die Dimensionierung der Nasen erfolgt bevorzugt im Verhältnis zum Durchmesser des Spritzenkörpers. Bevorzugt weist die erste und die zweite Nase eine radial bemessene Höhe von weniger als 50 %, vorzugsweise weniger als 35 %, insbesondere zwischen 5 % und 25 % eines Durchmessers des ersten Innenraums des Spritzenkörpers auf. Besonders bevorzugt beträgt die radial bemessene Höhe der Nase ungefähr 20 % des Durchmessers des Innenraums des Spritzenkörpers. Zur Optimierung der Nasenhöhen ist zu beachten, dass mit zunehmender Höhe der Verschwenkungsweg der länglichen Abschnitte vergrössert wird und bei abnehmender Höhe das Zurückhalten des Feststoffes möglicherweise nicht mehr gewährleistet werden kann. Die obigen Grössen haben sich als optimal herausgestellt. Bevorzugt weisen die erste und die zweite Nase eine radial bemessene Höhe von ungefähr 0.3 mm bei einem Innendurchmesser des Spritzenkörpers von ungefähr 1.6 mm auf.

Alternativ, insbesondere in Abhängigkeit des zu Injizierenden Feststoffes kann die Dimensionierung der Nase auch anders gewählt werden. Falls nur ein länglicher Abschnitt eingesetzt wird, können die Nasen auch eine grössere Höhe als 50 % des Durchmessers des Innenraums des Spritzenkörpers betragen. Insbesondere die erste Nase könnte auch so ausgebildet sein, dass sie den ersten Innenraum im Wesentlichen abschliesst. Entsprechend wird der Verschwenkungsweg der länglichen Abschnitte vergrössert. Bevorzugt umfasst der Stössel einen Kolben, wobei der Kolben eine Länge aufweist, welche grösser ist als der Abstand zwischen der ersten und der zweiten Nase. Damit wird erreicht, dass der Feststoff zwischen den Nasen gehalten werden kann. So kann der Feststoff, abgesehen von seiner eigenen Gewichtskraft, ohne Kraftbeaufschlagung an einem Hinausrutschen aus dem Spritzenkörper gehindert werden.

Alternativ kann der Abstand auch kleiner bemessen sein. In diesem Fall würde der Feststoff durch eine Reibungskraft zwischen der Nase (oder den Nasen) und dem Feststoff gehalten werden. Die beaufschlagte Kraft könnte dabei bei geeigneter Oberfläche der Nase und/oder des Feststoffes relativ klein bemessen sein, so dass sich die Vorteile der schonenden Handhabung des Feststoffes trotzdem ergeben würde, insbesondere da der Feststoff erst bei deaktivierter Haltevorrichtung durch den Stössel verschoben werden würde. Die Nase könnte dazu eine Oberflächenstruktur aufweisen, welche einen besonders hohen Reibungskoeffizient zwischen dem Feststoff und der Nase bildet. Weiter könnte die Nase auch eine geeignete Beschichtung aufweisen (z.B. Gummi), respektive aus einem geeigneten Material gebildet sein.

Der Stössel weist bevorzugt Ausnehmungen auf, welche mit der ersten und/oder der zweiten Nase zusammenwirken und ein Zurückziehen des Stössels in proximaler Richtung verhindern. Dies kann zum Beispiel dadurch erreicht werden, dass der Stössel quer zur Längsrichtung ausgerichtete Rillen aufweist und die zweite Nase keilförmig ausgebildet ist, so dass die zweite Nase in Eingriff mit den Rillen stehen kann. Dadurch, dass die Abschrägung der zweiten Nase in proximaler Richtung orientiert und die distale Seite der keilförmigen Nase radial, das heisst rechtwinklig zur Längsrichtung orientiert ist, kann der Stössel nur in distaler Richtung verfahren werden. Um diesen Effekt zu optimieren können die Rillen als keilförmige Ausnehmungen ausgebildet sein, wobei eine rechtwinklig zur Längsrichtung orientierte Seite distal angeordnet ist.

Alternativ kann auch auf eine obig beschriebene Ausbildung des Stössels verzichtet werden, insbesondere, wenn die Spritze für einen mehrfachen Gebrauch vorgesehen ist.

Vorzugsweise ist der Spritzenkörper aus einem federnden Material, insbesondere aus einem Metall, einer Metalllegierung oder einem Kunststoff, ausgebildet. Damit wird eine einstückige Ausbildung des Spritzenkörpers mit der Rückhaltevorrichtung ermöglicht. Dabei sind die Anforderungen an die elastische Eigenschaft des Materials nicht ausgesprochen hoch. Die Verschwenkung des federnden Elements ist typischerweise relativ gering. So beträgt der Verschwenkungswinkel des länglichen Abschnittes bevorzugt weniger als 10°, vorzugsweise weniger als 5°, insbesondere weniger als 2°.

Alternativ kann der Spritzenkörper auch aus einem nicht federnden Material ausgebildet sein, insbesondere, wenn die Rückhaltevorrichtung nicht einstückig mit dem Spritzenkörper ausgebildet wird.

Der Spritzenkörper ist bevorzugt aus einem transparenten Kunststoff ausgebildet. Damit wird erreicht, dass der Verwender visuell den Verabreichungsstatus des Feststoffes kontrollieren kann. Als Kunststoff können zum Beispiel Polymethylmethacrylat (PMMA), Polycarbonat oder weitere dem Fachmann bekannte Kunststoffe verwendet werden. Obschon PMMA relativ spröde und nur geringfügig elastisch ist, kann dieser Kunststoff für die Herstellung des Spritzenkörpers verwendet werden, insbesondere da an die elastische Eigenschaft keine hohen Anforderungen gestellt sind.

Alternativ können auch nicht transparente Materialien verwendet werden. Es ist auch denkbar, ein Sichtfenster im Spritzenkörper vorzusehen, welches aus einem anderen Material als der Spritzenkörper besteht.

Dem Fachmann ist jedoch eine Vielzahl von geeigneten Kunststoffen bekannt. So könnten alternativ auch andere geeignete Kunststoffe verwendet werden, welche eine gewisse Elastizität und dabei eine genügend hohe Festigkeit aufweisen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine schematische Darstellung einer erfindungsgemässen Vorrichtung zur Injektion eines Feststoffes;
- Fig. 2: eine schematische Darstellung eines Schnitts entlang einer Längsachse der Vorrichtung zur Injektion eines Feststoffes vor der Verwendung;
- Fig. 3: eine schematische Darstellung eines Schnitts entlang einer Längsachse der Vorrichtung zur Injektion eines Feststoffes bei deaktivierter Rückhaltevorrichtung;
- Fig. 4: eine schematische Darstellung eines Schnitts entlang einer Längsachse der Vorrichtung zur Injektion eines Feststoffes bei deaktivierter Rückhaltevorrichtung und in der Kanüle platzierten Feststoff;
- Fig. 5: eine schematische Darstellung eines Schnitts entlang einer Längsachse einer weiteren möglichen Ausführungsform einer erfindungsgemässen Vorrichtung zur Injektion eines Feststoffes eingeführt in einen Körper, vor der Betätigung des Stössels;
- Fig. 6: eine schematische Darstellung eines Schnitts entlang einer Längsachse der Vorrichtung zur Injektion eines Feststoffes, bei deaktivierter Rückhaltevorrichtung und in der Kanüle platziertem Feststoff, welche in einen Körper eingeführt ist; und
- Fig. 7: eine schematische Darstellung eines Schnitts entlang einer Längsachse der Vorrichtung zur Injektion eines Feststoffes nach der Platzierung des Feststoffes im Körper.

Grundsätzlich sind in den Figuren gleiche Teile mit gleichen Bezugszeichen versehen.

### Wege zur Ausführung der Erfindung

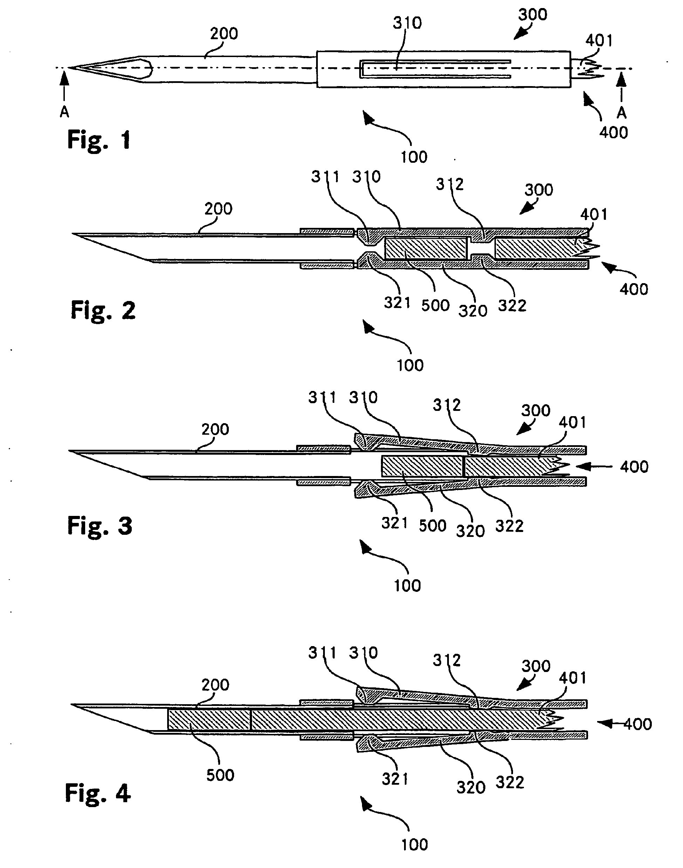

Die Figur 1 zeigt eine schematische Darstellung einer Vorrichtung 100 zur Injektion eines Feststoffes 500, welche eine Kanüle 200, einen Spritzenkörper 300 und einen Stössel 400 umfasst. Die Kanüle 200 weist an einem distalen Ende eine Abschrägung auf, womit ein Einführen in einen Körper vereinfacht wird. Der Spritzenkörper 300 ist im Wesentlichen als Kreiszylinder ausgebildet. Entlang einer Längsachse weist der Spritzenkörper zwei gegenüberliegende U-förmige Ausnehmungen auf, welche einen länglichen, federnden Abschnitt 310 einschliessen, wobei in der Figur 1 nur eine U-förmige Ausnehmung ersichtlich ist. Die Kanüle 200 ist über ein proximales Ende mit dem Spritzenkörper 300 verbunden. Der Stössel 400 umfasst einen Kolben 401 und einen Stösselkopf (nicht dargestellt). Der Kolben 401 weist im Wesentlichen die Form eines Kreiszylinders auf. Die Innenräume des Spritzenkörpers 300 und der Kanüle 200 weisen eine entsprechende Form auf, so dass der Stössel 400 durch den Spritzenkörper 300 und die Kanüle 200 hindurch verfahren werden kann. Der Spritzenkörper 300 kann eine Schutzhülle (nicht dargestellt) umfassen, welche ein Eindringen von Verunreinigungen bei der U-förmigen Ausnehmung verhindern kann. Die Schutzhülle ist entweder flexibel ausgebildet oder weist im Bereich der länglichen Abschnitte 310, 320 Ausnehmungen auf, so dass das Verschwenken der länglichen Abschnitte 310, 320 nicht behindert wird.

Der Spritzenkörper 300 und der Stössel 400 sind aus einem Kunststoff, insbesondere aus einem transparenten Kunststoff, zum Beispiel aus Polymethylmethacrylat (PMMA) oder Polycarbonat ausgebildet. Die Kanüle 200 besteht aus Metall, insbesondere aus Stahl.

Die Figur 2 zeigt eine schematische Darstellung eines Schnitts entlang der Achse A-A gemäss Figur 1 einer Vorrichtung 100 zur Injektion eines Feststoffes 500 vor der Verwendung. Dabei ist die Kanüle 200 ersichtlich, welche mit dem Spritzenkörper 300 verbunden ist. Der Spritzenkörper 300 umfasst in der vorliegenden Ausführungsform zwei einander gegenüberliegende längliche Abschnitte 310; 320, welche jeweils eine erste Nase 311; 321 und eine zweite Nase 312; 322 umfassen, welche in den Innenraum des Spritzenkörpers 300 hineinragen. Die länglichen Abschnitte 310; 320 sind parallel zur Längsachse des Spritzenkörpers 300 angeordnet und über das proximale Ende federnd und schwenkbar im Spritzenkörper 300 verbunden. In einem kraftlosen Zustand sind die länglichen Abschnitte 310; 320 parallel zur Längsachse orientiert. Die ersten Nasen 311; 321 sind an einem distalen Ende des entsprechenden länglichen Abschnittes 310; 320 mit demselben verbunden. Die zweiten Nasen 312; 322 sind im Bereich des proximalen Endes des entsprechenden länglichen Abschnittes 310; 320 mit demselben verbunden. Der Feststoff 500 hat die Form eines Kreiszylinders, welcher einen Durchmesser im Bereich des Durchmessers des Kolbens 401 aufweist. Der Abstand zwischen den ersten Nasen 311; 321 und den zweiten Nasen 312; 322 ist in der vorliegenden Ausführungsform geringfügig grösser als die Länge des Feststoffes 500, so dass der Feststoff zwischen den ersten Nasen 311; 321 und den zweiten Nasen 312; 322 formschlüssig gehalten und dadurch an einer Bewegung in eine Längsrichtung gehindert ist. Die zweiten Nasen 312; 322 sind bezüglich der Verbindung im Spritzenkörper 300 distal beabstandet angeordnet, so dass bei einer Kontaktierung des Stössels 400 bei der zweiten Nase 312; 322 eine Hebelwirkung erzielt werden kann. Die zweiten Nasen 312; 322 weisen dazu eine Abschrägung in proximaler Richtung auf. Beim distalen Verfahren des Stössels 400 kontaktiert nun der Stössel 400 die Abschrägung der zweiten Nasen 312; 322, um diese radial nach aussen zu treiben.

Die Figur 3 zeigt eine schematische Darstellung eines Schnitts entlang einer Längsachse einer Vorrichtung 100 zur Injektion eines Feststoffes 500 bei deaktivierter Rückhaltevorrichtung, wobei dieselben Elemente wie in der Figur 2 ersichtlich sind. Der Stössel 400 ist in distaler Richtung vorgerückt. Die Mantelfläche des Stössels 400 kontaktiert die zweiten Nasen 312; 322, so dass diese, gegenüber der Figur 2 radial nach aussen verschwenkt werden. Die über den länglichen Abschnitt 310; 320 mit den zweiten Nasen 312; 322 verbundenen ersten Nasen 311; 321 werden gleichzeitig, über Femwirkung, mittels Interaktion der zweiten Nasen 312; 322 und des Kolbens 401 radial nach aussen geführt, womit die ersten Nasen 311; 321 nicht mehr (oder höchstens teilweise) in den Innenraum des Spritzenkörpers 300 hineinragen und so den Weg für den Festsoff 500 durch den Innenraum des Spritzenkörpers 300 hindurch in die Kanüle 200 freigeben.

Die Figur 4 zeigt eine schematische Darstellung eines Schnitts entlang einer Längsachse einer Vorrichtung 100 zu Injektion eines Feststoffes 500 bei deaktivierter Rückhaltevorrichtung und in der Kanüle platziertem Feststoff. Die ersichtlichen Elemente entsprechen wiederum denjenigen der Figuren 2 und 3, wobei der Kolben 401 des Stössels 400 in die Kanüle 200 vorgerückt ist, womit der Feststoff 500 in der Kanüle 200 zu liegen kommt.

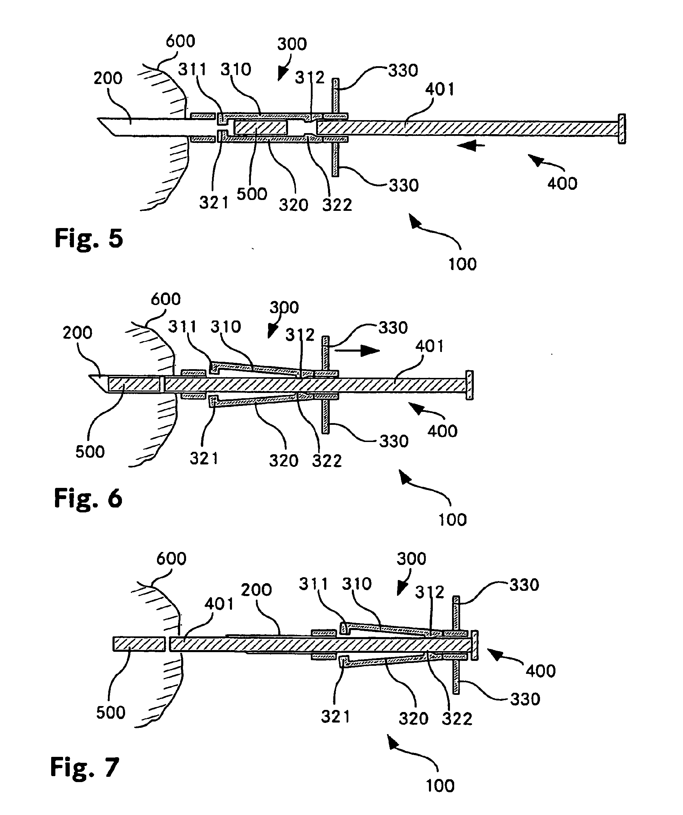

Die Figur 5 zeigt eine schematische Darstellung eines Schnitts entlang einer Längsachse einer Vorrichtung 100 zu Injektion eines Feststoffes 500, eingeführt in einen Körper 600 im Zustand vor der Betätigung des Stössels 400. Die ersichtlichen Elemente entsprechen weitgehend denjenigen der Figuren 2 - 4. Im Unterschied zu den vorhergehenden Figuren zeigt die Figur 5 weiter ein Haltelement 330, welches im proximalen Bereich des Spritzenkörpers 300 mit demselben verbunden ist. Das Halteelement 330 ist in der vorliegenden Ausführungsform umlaufend ausgebildet und kann zum Beispiel eine ovale oder nierenförmige Form aufweisen.

Die Kanüle 200 ist bis zum distalen Ende des Spritzenkörpers 300 in einen Körper 600 (eines Tiers oder eines Menschen) eingeführt. Die Vorrichtung 100 ist damit für die Injektion bereit. In einem nächsten Schritt wird nun der Stössel 400 in den Spritzenkörper verfahren, wobei der Stössel 400 bei den zweiten Nasen 312, 322 die länglichen Abschnitte 310, 320 radial nach aussen treibt, womit die ersten Nasen 311, 321 proportional zur radialen Bewegung der zweiten Nasen 312, 322 auch radial nach aussen getrieben werden. Dadurch wird der Weg für den Feststoff 500 durch den Spritzenkörper 300 hindurch in die Kanüle 200 freigegeben. Dabei ist zu beachten, dass der Kolben 401 den Feststoff 500 nicht in den Körper, sondern lediglich in einen distalen Bereich der Kanüle 200 befördert, welcher sich im Körper befindet. Damit wird eine Beschädigung des Körpergewebes durch den meist stumpfen Feststoff 500 verhindert und es werden möglichst wenig Schmerzen verursacht.

Die Figur 6 zeigt eine schematische Darstellung eines Schnitts entlang einer Längsachse einer Vorrichtung 100 zur Injektion eines Feststoffes 500 gemäss der Figur 5. Die Rückhaltevorrichtung ist deaktiviert und der Feststoff 500 ist bereits durch den Kolben 401 in der Kanüle, welche sich innerhalb des Körpers 600 befindet, platziert. Der Feststoff 500 ragt insbesondere nicht aus der Kanüle 200 hinaus, so dass das Gewebe des Körpers 600 nicht durch den meist stumpfen Feststoff beschädigt wird. Die länglichen Abschnitte 310, 320 sind radial nach aussen verschwenkt. Der Kolben 401 ist in diesem Zustand nicht vollständig in den Spritzenkörper 300 eingefahren, sondern ragt mit mindestens einer Länge, welche der Eindringtiefe der Kanüle 200 im Körper entspricht, aus dem proximalen Ende des Spritzenkörpers 300 heraus. Die exakte Länge, um welche der Stössel 400 in den Spritzenkörper 300 eingefahren werden soll kann beispielsweise lediglich auf dem Kolben 401 markiert sein, oder als Einkerbung im Kolben 401 realisiert sein, so dass eine reversible Einrastung einer oder beider zweiten Nasen 312, 322 erfolgt, sobald der Stössel 400 genügend weit in die Kanüle 200, respektive durch den Spritzenkörper 300 hindurch gefahren ist.

In einem nächsten Schritt wird der Spritzenkörper am Halteelement 330 ergriffen und in proximaler Richtung über den bezüglich des Körpers 600 ortsfesten Stössels 400 zurückgeschoben, bis die Kanüle 200 über den Feststoff 500 hinweggefahren ist und somit den Feststoff 500 im Körper 600 hinterlässt. Dadurch, dass der Stössel 400 während des Zurückziehens des Spritzenkörpers 300 ortsfest bleibt, wird erreicht, dass der Feststoff 500 während dem Hinausziehen der Kanüle 200 nicht durch Reibkräfte, hervorgerufen durch die Innenwandung der Kanüle 200, wieder aus dem Körper zurückgezogen wird.

Die Figur 7 zeigt eine schematische Darstellung eines Schnitts entlang einer Längsachse einer Vorrichtung 100 zur Injektion eines Feststoffes 500 nach der Platzierung des Feststoffes 500 im Körper 600. Der Spritzenkörper 300 ist bezüglich des Stössels 400 vollständig zurückgezogen, so dass die Kanüle 200 nicht mehr in den Körper 600 eindringt. Der Kolben 401 befindet sich in diesem Zustand noch im Körper 600. In einem letzten Schritt wird die Vorrichtung 100 vom Körper 600 zurückgezogen, so dass das distale Ende des Kolbens 401 aus dem Körper 600 entfernt wird.

Die Dimensionen der Vorrichtung 100 können grundsätzlich beliebig gewählt, insbesondere dem zu injizierenden Feststoff 500 angepasst werden.

Der Feststoff 500 kann auch eine andere Form aufweisen, ohne dass die Funktion der Vorrichtung beeinträchtigt würde. Der Feststoff 500 kann zum Beispiel die Form einer Pille oder einer Kugel aufweisen. Auch weitere Formen sind denkbar. Vorzugsweise kann aber die kleinste geometrische Projektion des Feststoffes in die Querschnittsfläche des Spritzenkörperinnenraums einbeschrieben werden.

Der Abstand zwischen den ersten 311; 321 und den zweiten Nasen 312; 322 kann auch kleiner als eine Länge des Feststoffes sein, womit der Feststoff mittels Reibschluss zwischen den ersten Nasen 311, 321 gehalten wird.

Auf die Abschrägung der zweiten Nasen 312; 322 kann auch verzichtet werden, insbesondere wenn der Kolben 401 am distalen Ende verrundet ist, oder entsprechend in distaler Richtung einen sich verringernden Querschnitt, respektive Abschrägungen aufweist.

Das Haltelement 330 kann auch zwei oder mehrteilig ausgebildet sein. Zum Beispiel könnten zwei gegenüberliegende Elemente vorgesehen sein, so dass der Spritzenkörper 300 zwischen Zeige- und Mittelfinger gehalten und mittels des Daumens der Stössel 400 betätigt werden kann.

Zusammenfassend ist festzustellen, dass durch die Erfindung eine Vorrichtung zum Injizieren eines Feststoffes geschaffen wird, welche ein feststoffschonendes Injizieren eines Feststoffes ermöglicht.

## Patentansprüche

1. Vorrichtung (100) zum Injizieren eines Feststoffes (500) in einen menschlichen oder einen tierischen Körper, insbesondere eine Spritze für die Injizierung eines Feststofimedikaments, umfassend:
a) einen Spritzenkörper (300) mit einem ersten rohrförmigen Innenraum zur Aufnahme des Feststoffes (500);
b) eine Rückhaltevorrichtung zum Zurückhalten des Feststoffes (500) im Spritzenkörper (300);
c) eine Kanüle (200) mit einem zweiten rohrförmigen Innenraum, wobei die Kanüle (200) mit dem Spritzenkörper (300) verbunden ist, und wobei der zweite rohrförmige Innenraum der Kanüle (200) mit dem ersten rohrförmigen Innenraum des Spritzenkörpers (300) kommuniziert;
d) einen Stössel (400), welcher durch den ersten Innenraum hindurch in den zweiten Innenraum verfahrbar ist; wobei
e) der Stössel (400) direkt mit der Rückhaltevorrichtung zusammenwirkbar ausgebildet ist, so dass die Rückhaltevorrichtung mittels des Stössels (400) deaktivierbar ist;und wobei
f) die Rückhaltevorrichtung ein Rückhalteelement (311; 321) umfasst, wobei der Stössel (400) bei der Deaktivierung der Rückhaltevorrichtung vom Rückhalteelement (311; 321) beabstandet ist; und wobei
g) das Rückhalteelement (311; 321) als mindestens eine in den ersten Innenraum des Spritzenkörpers (300) eindringende erste Nase (311; 321) ausgebildet ist, **dadurch gekennzeichnet, dass**
h) die erste Nase (311; 321) federnd mit dem Spritzenkörper (300) verbunden ist.

2. Vorrichtung (100) nach Anspruch 1, **dadurch gekennzeichnet, dass** der Feststoff (500) vor der Deaktivierung der Rückhaltevorrichtung zwischen dem Stössel (400) und dem Rückhalteelement (311; 321) gehalten ist.

3. Vorrichtung (100) nach Anspruch 1 oder 2, **dadurch gekennzeichnet, dass** die Rückhaltevorrichtung einstückig mit dem Spritzenkörper (300) ausgebildet ist.

4. Vorrichtung (100) nach Anspruch 1, **dadurch gekennzeichnet, dass** die Rückhaltevorrichtung einen länglichen Abschnitt (310; 320) umfasst, welcher an einem distalen Ende mit der ersten Nase (311; 321) verbunden ist und an einem proximalen Ende insbesondere federnd und/oder verschwenkbar mit dem Spritzenkörper (300) verbunden ist, wobei In einem ersten Zustand die Nase (311; 321) in den ersten Innenraum des Spritzenkörpers (300) eindringt und in einem zweiten Zustand höchstens teilweise in den ersten Innenraum des Spritzenkörpers (300) eindringt, insbesondere nicht in den ersten Innenraum des Spritzenkörpers (300) eindringt.

5. Vorrichtung (100) nach Anspruch 4, **dadurch gekennzeichnet, dass** die Rückhaltevorrichtung zwei bezüglich des ersten Innenraums gegenüberliegende längliche Abschnitte umfasst (310; 320), welche am distalen Ende mit Nasen (311; 321) verbunden sind und wobei die länglichen Abschnitte (310; 320) am proximalen Ende insbesondere federnd und/oder verschwenkbar mit dem Spritzenkörper (300) verbunden sind.

6. Vorrichtung (100) nach Anspruch 4 oder 5, **dadurch gekennzeichnet, dass** der längliche Abschnitt (310; 320) zwischen einer in Längsrichtung orientierten U-förmigen Ausnehmung im Spritzenkörper (300) gebildet ist.

7. Vorrichtung (100) nach einem der Ansprüche 4 - 6, **dadurch gekennzeichnet, dass** der längliche Abschnitt (310; 320) im ersten Zustand im Wesentlichen parallel zu einer Längsrichtung des ersten Innenraums des Spritzenkörpers (300) orientiert ist und im zweiten Zustand zur Längsrichtung des ersten Innenraums verschwenkt orientiert ist.

8. Vorrichtung (100) nach einem der Ansprüche 4 - 7, **dadurch gekennzeichnet, dass** der längliche Abschnitt (310; 320) im Bereich des proximalen Endes eine zweite Nase (312; 322) aufweist, welche im ersten Zustand in den Innenraum des Spritzenkörpers (300) eindringt und im zweiten Zustand höchstens teilweise in den ersten Innenraum des Spritzenkörpers (300) eindringt, insbesondere nicht in den ersten Innenraum des Spritzenkörpers (300) eindringt, wobei der Feststoff (500) im ersten Zustand zwischen der ersten Nase (311; 321) und der zweiten Nase (312; 322) zurückgehalten ist und im zweiten Zustand nicht zurückgehalten ist.

9. Vorrichtung (100) nach Anspruch 8, **dadurch gekennzeichnet, dass** der Stössel (400) und/oder die zweite Nase (312; 322) in proximaler Richtung eine Abschrägung aufweisen, so dass bei einem Zusammenwirken des Stössels (400) mit der zweiten Nase (312; 322) zumindest eine Kraftkomponente radial auf die zweite Nase beaufschlagbar und der längliche Abschnitt (310; 320) in den zweiten Zustand überführbar ist.

10. Vorrichtung (100) nach Anspruch 8 oder 9, **dadurch gekennzeichnet, dass** eine radial bemessene Höhe der ersten Nase (311; 321) grösser als eine radial bemessene Höhe der zweiten Nase (312; 322) ist.

11. Vorrichtung (100) nach dem Anspruch 10, **dadurch gekennzeichnet, dass** ein Verhältnis zwischen der radial bemessenen Höhe der ersten Nase (311; 321) zu einem Abstand der ersten Nase zum proximalen Ende des länglichen Abschnittes (310; 320) in einem Bereich eines Verhältnisses zwischen der radial bemessenen Höhe der zweiten Nase (312; 322) zu einem Abstand der zweiten Nase (312; 322) zum proximalen Ende des länglichen Abschnittes (310; 320) liegt.

12. Vorrichtung (100) nach einem der Ansprüche 8 - 11, **dadurch gekennzeichnet, dass** die erste (311; 321) und die zweite Nase (312; 322) eine radial bemessene Höhe weniger als 50 %, vorzugsweise weniger als 35 %, insbesondere zwischen 5 % und 25 % eines Durchmessers des ersten Innenraums des Spritzenkörpers (300) aufweisen.

13. Vorrichtung (100) nach einem der Ansprüche 8 - 12, **dadurch gekennzeichnet, dass** der Stössel (400) einen Kolben (401) umfasst, wobei der Kolben (401) eine Länge aufweist, welche grösser ist als der Abstand zwischen der ersten (311; 321) und der zweiten Nase (312; 322).

14. Vorrichtung (100) nach einem der Ansprüche 8 - 13, **dadurch gekennzeichnet, dass** der Stössel (400) Ausnehmungen aufweist, welche mit der ersten (311; 321) und/oder der zweiten Nase (312; 322) zusammenwirken und ein Zurückziehen des Stössels (400) in proximaler Richtung verhindern.

15. Vorrichtung (100) nach einem der Ansprüche 1 - 14, **dadurch gekennzeichnet, dass** der Spritzenkörper (300) aus einem zumindest geringfügig federnden Material, insbesondere aus einem Metall, einer Metalllegierung oder einem Kunststoff, ausgebildet Ist.

16. Vorrichtung (100) nach einem der Ansprüche 1 - 15, **dadurch gekennzeichnet, dass** der Spritzenkörper (300) aus einem transparenten Kunststoff ausgebildet ist.

## Claims

1. Device (100) for injecting a solid (500) into a human or animal body, in particular a syringe for injecting a solid medicament, said device (100) comprising:
a) a syringe body (300) with a first tubular interior for receiving the solid (500);
b) a retaining device for retaining the solid (500) in the syringe body (300);
c) a needle (200) with a second tubular interior, said needle (200) being connected to the syringe body (300), and the second tubular interior of the needle (200) communicating with the first tubular interior of the syringe body (300);
d) a ram (400), which can be driven through the first interior into the second interior; wherein
e) the ram (400) is designed to cooperate directly with the retaining device, such that the retaining device can be deactivated by means of the ram (400); and ;and wherein
f) the retaining device comprises a retaining element (311; 321), the ram (400) being at a distance from the retaining element (311; 321) upon deactivation of the retaining device; and wherein
g) the retaining element (311; 321) is designed as at least a first lug (311; 321) extending into the first interior of the syringe body (300),
**characterized in that**
h) the first lug (311; 321) is connected resiliently to the syringe body (300).

2. Device (100) according to Claim 1, **characterized in that** the solid (500) is held between the ram (400) and the retaining element (311; 321) prior to the deactivation of the retaining device.

3. Device (100) according to Claim 1 or 2, **characterized in that** the retaining device is formed in one piece with the syringe body (300).

4. Device (100) according to Claim 1, **characterized in that** the retaining device comprises an elongate portion (310; 320) which, at a distal end, is connected to the first lug (311; 321) and, at a proximal end, is connected to the syringe body (300), in particular resiliently and/or pivotably, and the lug (311; 321), in a first state, extends into the first interior of the syringe body (300) and, in a second state, extends at most partially into the first interior of the syringe body (300) or, in particular, does not extend into the first interior of the syringe body (300).

5. Device (100) according to Claim 4, **characterized in that** the retaining device comprises two elongate portions (310; 320), which lie opposite each other in relation to the first interior and which are connected at the distal end to lugs (311; 321), and the elongate portions (310; 320) are connected at the proximal end to the syringe body (300), in particular resiliently and/or pivotably.

6. Device (100) according to Claim 4 or 5, **characterized in that** the elongate portion (310; 320) is formed between a longitudinally oriented U-shaped recess in the syringe body (300).

7. Device (100) according to one of Claims 4 - 6, **characterized in that** the elongate portion (310; 320), in the first state, is oriented substantially parallel to a longitudinal direction of the first interior of the syringe body (300) and, in the second state, is oriented in a pivoted position relative to the longitudinal direction of the first interior.

8. Device (100) according to one of Claims 4 - 7, **characterized in that** the elongate portion (310; 320) has, in the area of the proximal end, a second lug (312; 322) which, in the first state, extends into the interior of the syringe body (300) and, in the second state, extends at most partially into the first interior of the syringe body (300) or, in particular, does not extend into the first interior of the syringe body (300), and the solid (500), in the first state, is retained between the first lug (311; 321) and the second lug (312; 322) and, in the second state, is not retained.

9. Device (100) according to Claim 8, **characterized in that** the ram (400) and/or the second lug (312; 322) in the proximal direction have a bevel such that, when the ram (400) engages with the second lug (312; 322), at least one force component can be applied radially to the second lug, and the elongate portion (310; 320) can be converted to the second state.

10. Device (100) according to Claim 8 or 9, **characterized in that** a radially measured height of the first lug (311; 321) is greater than a radially measured height of the second lug (312; 322).

11. Device (100) according to Claims 10, **characterized in that** a ratio of the radially measured height of the first lug (311; 321) to a distance of the first lug from the proximal end of the elongate portion (310; 320) lies in a range of a ratio of the radially measured height of the second lug (312; 322) to a distance of the second lug (312; 322) from the proximal end of the elongate portion (310; 320).

12. Device (100) according to one of Claims 8 - 11, **characterized in that** the first lug (311; 321) and the second lug (312; 322) have a radially measured height that is less than 50%, preferably less than 35%, particularly between 5% and 25%, of a diameter of the first interior of the syringe body (300).

13. Device (100) according to one of Claims 8 - 12, **characterized in that** the ram (400) comprises a plunger (401), and the plunger (401) has a length that is greater than the distance between the first lug (311; 321) and the second lug (312; 322).

14. Device (100) according to one of Claims 8 - 13, **characterized in that** the ram (400) has recesses which cooperate with the first lug (311; 321) and/or the second lug (312; 322) and prevent the ram (400) from being pulled back in the proximal direction.

15. Device (100) according to one of Claims 1 - 14, **characterized in that** the syringe body (300) is made from an at least slightly resilient material, in particular a metal, a metal alloy or a plastic.

16. Device (100) according to one of Claims 1 - 15, **characterized in that** the syringe body (300) is made from a transparent plastic.

## Revendications

1. Dispositif (100) pour injecter une matière solide (500) dans un corps humain ou animal, en particulier seringue pour l'injection d'un médicament solide, comprenant:
a) un corps de seringue (300) avec un premier espace intérieur tubulaire destiné à contenir une matière solide (500);
b) un dispositif de retenue destiné à retenir la matière solide (500) dans le corps de seringue (300);
c) une canule (200) avec un deuxième espace intérieur tubulaire, dans lequel la canule (200) est reliée au corps de seringue (300), et dans lequel le deuxième espace intérieur tubulaire de la canule (200) communique avec le premier espace intérieur tubulaire du corps de seringue (300);
d) un poussoir (400), qui est déplaçable dans le deuxième espace intérieur à travers le premier espace intérieur; dans lequel
e) le poussoir (400) est apte à coopérer directement avec le dispositif de retenue, de telle manière que le dispositif de retenue puisse être désactivé au moyen du poussoir (400); et dans lequel
f) le dispositif de retenue comprend un élément de retenue (311; 321), dans lequel le poussoir (400) est espacé de l'élément de retenue (311; 321) lors de la désactivation du dispositif de retenue; et dans lequel
g) l'élément de retenue (311; 321) est réalisé sous la forme d'au moins un premier ergot (311; 321) pénétrant dans le premier espace intérieur du corps de seringue (300),
**caractérisé en ce que**
h) le premier ergot (311; 321) est relié de façon élastique au corps de seringue (300).

2. Dispositif (100) selon la revendication 1, **caractérisé en ce que** la matière solide (500) est maintenue entre le poussoir (400) et l'élément de retenue (311; 321) avant la désactivation du dispositif de retenue.

3. Dispositif (100) selon la revendication 1 ou 2, **caractérisé en ce que** le dispositif de retenue est réalisé d'une seule pièce avec le corps de seringue (300).

4. Dispositif (100) selon la revendication 1, **caractérisé en ce que** le dispositif de retenue comprend une section allongée (310; 320), qui est reliée par une extrémité distale au premier ergot (311; 321) et qui est reliée par une extrémité proximale au corps de seringue (300), en particulier de façon élastique et/ou pivotante, dans lequel, dans un premier état, l'ergot (311; 321) pénètre dans le premier espace intérieur du corps de seringue (300) et, dans un deuxième état, pénètre au maximum partiellement dans le premier espace intérieur du corps de seringue (300), en particulier ne pénètre pas dans le premier espace intérieur du corps de seringue (300).

5. Dispositif (100) selon la revendication 4, **caractérisé en ce que** le dispositif de retenue comprend deux sections allongées (310; 320) opposées l'une à l'autre par rapport au premier espace intérieur, qui sont reliées par l'extrémité distale a des ergots (311; 321) et dans lequel les sections allongées (310; 320) sont reliées par l'extrémité proximale au corps de seringue (300), en particulier de façon élastique et/ou pivotante.

6. Dispositif (100) selon la revendication 4 ou 5, **caractérisé en ce que** la section allongée (310; 320) est formée entre un évidement en forme de U orienté en direction longitudinale dans le corps de seringue (300).

7. Dispositif (100) selon l'une quelconque des revendications 4 6, **caractérisé en ce que** la section allongée (310; 320) est orientée, dans le premier état, sensiblement parallèlement à une direction longitudinale du premier espace intérieur du corps de seringue (300) et est orientée, dans le deuxième état, en position inclinée par rapport à la direction longitudinale du premier espace intérieur.

8. Dispositif (100) selon l'une quelconque des revendications 4 à 7, **caractérisé en ce que** la section allongée (310; 320) présente dans la région de l'extrémité proximale un deuxième ergot (312; 322) qui, dans le premier état, pénètre dans l'espace intérieur du corps de seringue (300) et, dans le deuxième état, pénètre au maximum partiellement dans le premier espace intérieur du corps de seringue (300), en particulier ne pénètre pas dans le premier espace intérieur du corps de seringue (300), dans lequel la matière solide (500) est retenue dans le premier état entre le premier ergot (311; 321) et le deuxième ergot (312; 322) et n'est pas retenue dans le deuxième état.

9. Dispositif (100) selon la revendication 8, **caractérisé en ce que** le poussoir (400) et/ou le deuxième ergot (312; 322) présentent dans la direction proximale un chanfrein, de telle manière que, lors d'une coopération du poussoir (400) avec le deuxième ergot (312; 322), au moins une composante de force puisse être appliquée radialement au deuxième ergot et que la section allongée (310; 320) puisse être amenée dans le deuxième état.

10. Dispositif (100) selon la revendication 8 ou 9, **caractérisé en ce qu'**une hauteur mesurée radialement du premier ergot (311; 321) est plus grande qu'une hauteur mesurée radialement du deuxième ergot (312; 322).

11. Dispositif (100) selon la revendication 10, **caractérisé en ce qu'**un rapport entre la hauteur mesurée radialement du premier ergot (311; 321) et une distance du premier ergot à l'extrémité proximale de la section allongée (310; 320) se situe dans une plage d'un rapport entre la hauteur mesurée radialement du deuxième ergot (312; 322) et une distance du deuxième ergot (312; 322) à l'extrémité proximale de la section allongée (310; 320).

12. Dispositif (100) selon l'une quelconque des revendications 8 à 11, **caractérisé en ce que** le premier ergot (311; 321) et le deuxième ergot (312; 322) présentent une hauteur mesurée radialement inférieure à 50 %, de préférence inférieure à 35 %, en particulier comprise entre 5 % et 25 %, d'un diamètre du premier espace intérieur du corps de seringue (300).

13. Dispositif (100) selon l'une quelconque des revendications 8 à 12, **caractérisé en ce que** le poussoir (400) comprend un piston (401), dans lequel le piston (401) présente une longueur qui est plus grande que la distance entre le premier ergot (311; 321) et le deuxième ergot (312; 322).

14. Dispositif (100) selon l'une quelconque des revendications 8 à 13, **caractérisé en ce que** le poussoir (400) présente des évidements, qui coopèrent avec le premier ergot (311; 321) et/ou avec le deuxième ergot (312; 322) et empêchent un retrait du poussoir (400) en direction proximale.

15. Dispositif (100) selon l'une quelconque des revendications 1 à 14, **caractérisé en ce que** le corps de seringue (300) est constitué d'un matériau au moins légèrement élastique, en particulier d'un métal, d'un alliage métallique ou d'une matière plastique.

16. Dispositif (100) selon l'une quelconque des revendications 1 à 15, **caractérisé en ce que** le corps de seringue (300) est réalisé en une matière plastique transparente.
